Europäisches Patentamt

European Patent Office

Office européen des brevets

(19)

(11) Publication number: **0 495 465 A2**

## EUROPEAN PATENT APPLICATION

(12)

(21) Application number: **92100528.6**

(22) Date of filing: **14.01.92**

(51) Int. Cl.⁵: **G01N 33/554**, G01N 33/569, G01N 33/571

(30) Priority: **15.01.91 US 641597**

(43) Date of publication of application:
**22.07.92 Bulletin 92/30**

(84) Designated Contracting States:
**AT BE CH DE DK ES FR GB GR IT LI LU NL PT SE**

(71) Applicant: **COULSTON INTERNATIONAL CORPORATION**
**2512 Christina Place**
**Alamagordo, New Mexico 88310(US)**

(72) Inventor: **Zeng, Yi, 16-2 Tower Building**

**Apart. of Chinese Academy of Traditional Medicine**
**Dongzhemon 100700, Beijing(CN)**
Inventor: **Wang, Zhe**
**2-404 Building, 41 No. Di An Mor Nei Street Beijing(CN)**
Inventor: **Coulston, Frederick**
**2512 Christina Place**
**Alamagordo, New Mexico 88310(CN)**

(74) Representative: **Hoeger, Stellrecht & Partner**
**Uhlandstrasse 14 c**
**W-7000 Stuttgart 1(DE)**

(54) **Immuno-enzymatic test for the detection of viral antibody.**

(57) An immuno-enzymatic method for the detection of antibodies to a virus under test, a diagnostic kit therefor, new cell lines for use in said method, a method for preparing said cell line for use in antibody detection, and an immuno-enzymatic test strip and kit therefor are described.

FIG. 1

EP 0 495 465 A2

## INTRODUCTION

The present invention relates to an immuno-enzymatic method for the detection of viral antibodies, and, more particularly, to an immuno-enzymatic method for the detection of human immunodeficiency virus antibody, a diagnostic kit therefor, new cell lines for use in said method, a method for preparing said cell lines for use in antibody detection, and an immuno-enzymatic test strip and kit therefor.

## BACKGROUND OF THE INVENTION

Methods to detect viruses that may have serious health implications are important not only for diagnosis and treatment, but also for epidemiology and the prevention of further infection of the virus within a given population.

With the advent of AIDS, viral testing has become a matter of grave importance. The AIDS virus is highly transmissible and ultimately can result in death of the infected individual. Because there is no cure for AIDS at present, the detection of AIDS-virus infected individuals is important in epidemiological studies and paramount in efforts to prevent the spread of the virus.

A major mode of viral transmission is through blood and its products. Therefore, it is important to identify potential blood donors who are infected with a virus. The only practical way to do this is to screen donors for the presence of antibodies.

Virus-based, enzyme-linked immuno-sorbent assays (ELISA) require special equipment and expertise and are very expensive to perform. In addition, they produce a significant number of false positive results. Immuno-fluorescence (IF) tests are less expensive and easier to perform. However, IF tests require the use of a fluorescence microscope. Therefore, the development of an accurate testing method that is inexpensive, easy to perform and does not require special equipment is desirable. Such a method would enable screening of drug addicts and other high risk individuals in public health facilities as well as testing of patients in doctors' offices, screening of laboratory animals, and consumer use.

## SUMMARY OF THE INVENTION

The present invention provides an immuno-enzymatic method for the detection of antibodies to a virus under test in a test sample, such as, in blood and blood components, novel cell lines specially prepared for use in the immuno-enzymatic method, and both diagnostic slide and test strip kits for carrying out the immuno-enzymatic method.

In accordance with one aspect of the invention, a novel cell line is incubated with the virus to be tested and, during incubation, the cells are activated with an inducer to promote accumulation of the virus, both within the cytoplasm of the cell and on the cell surface.

In accordance with another aspect of the invention, the activated, virus-infected cells are fixed to support means, incubated with a test sample, such as blood or components thereof, washed, incubated with a conjugate capable of binding to antibodies to the virus, washed, incubated with an indicator for the conjugate, washed, and then examined with a light microscope to observe a color change in the indicator. A color change is indicative of a positive test, i.e., it indicates the presence of antibodies to the virus under test in the test sample.

In yet another aspect of the invention, there is provided a test slide kit for the immuno-enzymatic detection of antibodies to a virus in a test sample.

In still another aspect of the invention, there is provided a test strip for the immuno-enzymatic detection of antibodies to a virus in a test sample.

In yet another and more specific aspect of the invention, there is provided a test strip for the immuno-enzymatic detection of HIV-1 antibody in a test sample, particularly blood or blood components, wherein the glycoprotein, gp41, is carried on the strip.

## BRIEF DESCRIPTION OF THE DRAWINGS

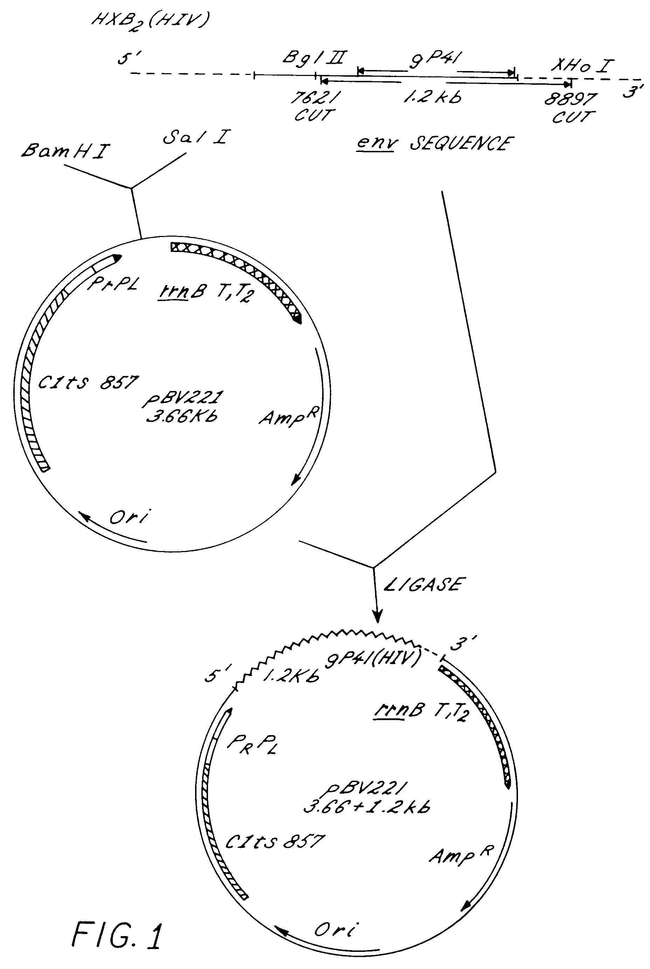

Figure 1 shows maps of the gene for gp41, the pBV221 expression vector and the recombinant pBV221 expression vector, containing the gene for gp41.

## DETAILED DESCRIPTION OF THE INVENTION

In general, compositions and methods of an immuno-enzymatic test for the detection of antibodies to a virus are provided. In accordance with the invention, there is provided an immuno-enzymatic method for the detection of viral antibodies in a test sample, such as blood or blood components. In one aspect of the invention, a cell line, innoculated with a virus of interest and activated with an inducer that promotes both intracellular and cell-surface accumulation of the virus, is used in the immuno-enzymatic detection method to increase the availability of the test virus for antibody binding, thereby providing a basis for a strong color reaction to take place. Detection of antibodies to the virus of interest in the test sample is thus enhanced.

In one preferred embodiment of the invention, CLL-8 cells were established from a human T-cell leukemia patient in China. The CLL-8 cells were cultivated and infected with HIV-1-AC virus, which was isolated from an American AIDS patient in China. In another preferred embodiment, CM-1 cells, a cell line established from T-cells of a comatose patient in China, were cultivated and infected with virus. Separately, large quantities of HIV-1-AC virus for infection of CLL-8 or CM-1 cells were obtained by infection of CEM cells (commercially available) with the virus.

The CLL-8 or CM-1 cells, infected with HIV-1-AC, are incubated for a period of time sufficient to allow growth of the virus in the cells. Thereafter the infected cells are activated with an inducer. It will be appreciated that the incubation period, after infection and prior to activation, must be sufficient to allow growth of the virus in the cells. However, the incubation period must not be so long that the infected cells are either destroyed or no longer suitable for cell smears. Activation of HIV-1-AC-infected CLL-8 cells after five days of incubation was found to be suitable.

The infected cells, incubated for a sufficient period of time, are activated with an inducer to accumulate virus intracellularly and to present large quantities of viral antigens on the cell surface. The intracellular accumulation of virus promoted by the inducer is much greater than that which would occur naturally. The preferred inducer comprises cortisone, 12-0-tetradecanoyl-phorbol-12-acetate (TPA) and butyric acid in tissue culture medium. The inducer may comprise 5 ng/ml cortisone, 5-20 ng/ml TPA and 1-4 mM butyric acid. In the preferred embodiment, infected CLL-8 or CM-1 cells were activated with an inducer consisting of 5 ng/ml cortisone, 5 ng/ml TPA and 2 mM butyric acid, in tissue culture medium. The preferred activation period is about 24 hours. Use of the preferred inducer provided the greatest accumulation of viral antigens, both intracellularly and on the cell surface, within the shortest period of time. The relative concentration of each inducer component may be either increased or decreased, as long as the period of activation is either decreased or increased, respectively. However, the period of activation should be such that the cells are not adversely affected.

It will be appreciated that the inducer can be used to induce viral accumulation in all T-cell leukemia cell lines including, for example, CEM, MT-4, HUT 78, and the like. The inducer also can be used to induce viral accumulation in B-cells, such as Raji cells.

Induced CLL-8 and CM-1 cells are especially suited for use in an immuno-enzymatic test because of the relatively high accumulation of virus, both intracellulary and on the cell surface. Because of this relatively high concentration of virus, subsequent treatment of these cells and addition of a test sample containing antibodies to the virus of interest results in the production of a detectable color change that is readily apparent by observation through a light microscope.

The virus-infected, induced CLL-8 or CM-1 cells are mixed with normal CLL-8 or CM-1 cells, respectively, for use in the immuno-enzymatic method. The mixing of normal and infected cells allows for the determination of nonspecific reaction. For example, if both infected and noninfected cells are stained, it can be inferred that antibodies in the test sample are binding to something other than or in addition to the virus of interest. Therefore, the mixing of normal and infected cells is preferred to minimize false positive tests. It is important to mix the cells in such a way as to allow for the determination of nonspecific reaction, yet allow for the generation of a color reaction that is easily observable. To that end, normal and infected cells may be mixed to make the positive rate 5-80%. A positive rate of 30-50%, however, is preferred. In carrying out the method, mixed cells are washed twice, centrifuged, and resuspended to a concentration of $3 \times 10^5$ cells/ml of RPMI-1640 to remove components in the test sample that may cause a false positive test result.

Resuspended cells are then placed onto a support, such as a slide or the like and allowed to dry. After drying, the cells are fixed with a fixative agent, such as acetone, for example. Acetone dissolves the lipids in cell membranes, thereby allowing HIV-1-specific antibodies, if present in a test sample, to react with HIV-1 antigens present inside the cells and on the cell membranes.

The fixed cell slide is incubated with a test sample, such as, for example, dilute blood or blood components. After incubation with the test sample, the slide is washed several times and incubated with a conjugate capable of binding to antibodies of the virus for which the test sample is being tested. The conjugate must also be capable of reacting with an indicator to cause a color change in the indicator. Any conjugate and indicator pair that result in color change detectable with a light microscope may be used. For example, conjugates that may be used with the indicator 3-amino-9-ethylcarbazole (AEC) include horseradish peroxidase (HRPO)-Staphylococcus protein A (SPA), HRPO-anti-human IgG antibody, alkaline phosphatase-SPA, and alkaline phosphatase-anti-human IgG antibody. In the preferred embodiment, the conjugate is HRPO-conjugated SPA. The HRPO-conjugated SPA binds to antibodies from the test sample that have bound to the virus of interest. Then the slide is washed again, and AEC indicator is added. In the presence of active HRPO, bound to the antibodies by conjugated SPA, AEC changes from colorless to a pink/red color. After final washing, the slide is examined under a light microscope. Pink coloration of cell membrane and cytoplasm indicates the presence of viral antibodies.

In another aspect of the invention, the immuno-enzymatic test is provided as a kit. The kit comprises multi-welled antigen slides to which induced, virus-infected CLL-8 or CM-1 cells have been fixed, and ampules for the wash solutions, indicator and conjugate necessary to carry out the test procedure described above. The test kit may be used for HIV-1, HIV-2, HTLV-1 and SIV-III viruses. Test kits differ only in the test virus, i.e., the virus used to infect the CLL-8 or CM-1 cells fixed to the wells on the antigen slides. Alternatively, B95-8 cells or Raji cells infected with Epstein-Barr virus or Vero cells infected with Ebola virus are used in test kits for the detection of Epstein-Barr or Ebola virus, respectively. The Raji cells preferably are activated with an inducer.

In another embodiment, the present invention provides a test strip for use in the immuno-enzymatic detection of the AIDS-virus antibody in a test sample. In this embodiment, the test strip includes a support means to which is affixed an adsorbent material. The adsorbent material is adsorbed with HIV-1 antigenic protein, glycoprotein gp41, for the detection of HIV-1 antibody.

In the preferred embodiment of this aspect of the invention, the HIV-1 gene for gp41 glycoprotein was obtained from the Chinese Academy of Traditional Medicine and cloned into the high efficiency expression vector, pBV221, which was then used to transform E. coli, all according to methods known to those skilled in the art (see, for example, Maniatis et al. 1982. Molecular Cloning: A Laboratory Manual, Cold Spring Harbor Laboratory) and as indicated, in part, in Figure 1. The cultured, transformed E. coli were disrupted by ultrasound and the insoluble parts, including proteins and broken bacteria, were precipitated. Then the precipitate was dissolved in 8M urea, the insoluble parts precipitated, and the resulting supernatant was dialysed to remove the urea and fractionated on a 15% polyacrylamide gel, making use of a stacking gel, to obtain pure gp41 protein (see, for example, Laemlli et al., J. Molec. Bio. 80:575, 1973). After electrophoresis, the proteins were transferred to nitrocellulose and the band containing gp41 was identified, both by monoclonal antibody and by positive HIV-1 serum. Once identified, the region of the nitrocellulose to which gp41 adsorbed was trimmed well within the outer edges of gp41 adsorption. In this manner, highly pure gp41, without any other protein, is employed as the detection means of the test strip. The use of such high purity gp41 provides a highly sensitive and very accurate test strip.

The strip of nitrocellulose, adsorbed with gp41 thus obtained is cut into pieces and affixed to a suitable support means, such as plastic, by gluing with "Double Swallow" adhesive, for example, which is manufactured by Beijing Liangxiang.

HIV-1 antigen test strips also may be prepared with the whole AIDS virus or with virus-infected CLL-8 or CM-1 cells. Regardless of whether the virus, the virus-infected cells or the gp41 protein is used, the assay is relatively short, typically taking only about 30 minutes, and it is easy to use. The assay is very accurate and very sensitive as discussed in the Examples below.

In another aspect of the invention, there is provided a kit which includes an immuno-enzymatic test strip. The kit comprises several test strips, one for positive control, one for negative control and one for the test sample, as well as vials containing the wash solutions, conjugate and indicator necessary to perform the immuno-enzymatic test procedure in accordance with the procedure described for the test slide. It will be appreciated that regardless of whether the test strip carries gp41, the AIDS virus or virus-infected CLL-8 or CM-1 cells, the procedure for carrying out the tests includes contacting the test sample with the test strip, contacting the infected test strip with the conjugate, thereafter contacting the test strip with the indicator, and then determining whether there is a color change. Of course, test strips which carry either gp41 or the AIDS virus will be used to detect antibodies to the AIDS virus. Test strips which carry virus-infected CLL-8 or CM-1 cells will be used to detect antibodies to the infecting virus. Viruses which may be used to infect the CLL-8 or CM-1 cells for use in the test strip include HIV-1, HIV-2, HTLV-1, and SIV-III. B95-8 and Raji cells infected with Epstein-Barr virus and Vero cells infected with Ebola virus also may be used in test strips for detection of antibodies to the respective infecting virus.

The following examples are illustrative of the invention and are not intended to limit the scope of the invention.

Example 1

This Example illustrates the cultivation of CLL-8 cells.

The CLL-8 cell line was established from a human T-cell leukemia patient in China. Cells were cultivated in suspension with 90% RPMI-1640, a culture medium that is commercially available, and 10% fetal calf serum at 37°C with 5% $CO_2$ to a concentration of 5 x $10^5$ cells/ml. Cell suspensions were split twice a week.

CLL-8 cells were stored in 70% RPMI-1640, 10% dimethylsulfoxide (DMSO), and 20% fetal calf serum. Initially, after preparation of cells for storage, the cells were stored at -70°C overnight. The cells were then stored at -196°C.

Prior to use, a tube of cells was moved from -196°C to 37°C rapidly. Five mls of RPMI-1640 were added to the tube and the cells were centrifuged at 1,000 rpm for 10 minutes. The supernatant was then removed and 5 mls of RPMI-1640, containing 10% fetal calf serum, were added to the pellet of cells. The cells were then cultivated at 37°C with 5% $CO_2$.

## Example 2

This Example illustrates the cultivation of virus.

The virus HIV-1-AC was isolated from an American AIDS patient in China. CEM cells were infected with the virus. Having been derived from a T-cell leukemia patient and being susceptible to HIV infection, the CEM cells were cultivated like CLL-8 cells and were used to prepare large quantities of HIV-1-AC virus.

## Example 3

This Example illustrates the infection of CLL-8 cells with virus.

The CLL-8 cells cultivated in Example 1 were split and cultivated for two days. After the second day of cultivation, the cells were collected by centrifugation. Cell pellets were infected with the HIV-1-AC virus cultivated in Example 2 and put at 37°C for two hours. Then, RPMI-1640, containing 10% fetal calf serum, was added and incubation at 37°C was continued with 5% $CO_2$.

## Example 4

This Example illustrates cell activation of the infected CLL-8 cells of Example 3 with an inducer.

An inducer was used to activate the infected CLL-8 cells of Example 3 to accumulate virus intracellularly and to present large quantities of viral antigens on the cell surface. The inducer consists of 5 ng/ml cortisone, 5 ng/ml TPA, and 2 mM butyric acid in culture medium.

The inducer was added to virus-infected CLL-8 cells in the fifth day after infection. About 24 hours after the inducer had been added, the cell culture was centrifuged at 2,000 rpm for 20 minutes. The supernatant was removed and the induced, virus-infected CLL-8 cells were collected.

The effectiveness of the inducer is illustrated in Table I. HIV-1-infected CLL-8 cells, activated in the fifth day after infection, were compared with noninduced, HIV-1-infected CLL-8 cells, both prior to and after the time of activation.

```
                      TABLE  I

                 Days  of  HVI-1  Infection
                 ( )  =  Days  of  Induction
                   5(0)          6(1)         7(2)

  CLL-8 Cells + Inducer      ++          ++++         ++++
  CLL-8 Cells - Inducer      ++       ++ or +++        +++


       ++  =  20%
      +++  =  50%
     ++++  =  80%
```

It can be seen from the data in Table I that the inducer was effective in promoting the accumulation of virus in the CLL-8 cells.

## Example 5

This Example illustrates the preparation of test slides using the activated HIV-1-infected CLL-8 cells of Example 4.

Normal and infected CLL-8 cells were collected and mixed to a positive rate of 30-50%, a preferred mixing rate. The mixed cells were washed twice with 0.01M PBS, pH 7.4, and centrifuged at 2,000 rpm for 15 minutes. The supernatant was removed and the cells were resuspended to a suitable concentration. Five ul of resuspended cells were placed into each of the 12 wells on a slide so as to form a cell monolayer in each well. The cells were then allowed to dry. After drying, the cells were fixed in the wells with cold acetone for 20 minutes. The prepared slides were then packaged with dessicant and stored at either 4°C or -20°C.

Example 6

This Example illustrates the determination of activity of HRPO-conjugated SPA which is to be used in carrying out the immuno-enzymatic test method.

Horseradish peroxidase (HRPO, commercially available) was prepared according to methods known to one of ordinary skill in the art. The HRPO was then conjugated with Staphylococcus protein A according to known methods.

The working density of the HRPO-conjugated SPA was determined by the greatest dilution of HRPO-conjugated SPA that responded to HIV-1-infected cells bound with IgG antibodies specific for the HIV-1 virus.

Smears of HIV-1 infected CLL-8 cells were incubated with serial PBS dilutions of a standard HIV-1 positive serum in a humidified atmosphere for 45 minutes at 37°C. Then, the smears of cells were washed three times with PBS for 5 minutes each wash. After washing, HRPO-conjugated SPA was added to the smears. The smears were incubated for an additional 45 minutes at 37°C and then washed three more times with PBS. Then, AEC indicator was added. In the presence of active HRPO, bound to antibodies by conjugation to SPA, AEC changes from colorless to a pink/red color. After final washing, the slides of cell smears were washed with PBS and the slides were examined under a light microscope. Pink coloration of cell membrane and cytoplasm indicates the presence of viral antibodies in the test sample.

Example 7

This Example illustrates the immuno-enzymatic test procedure using the activated, HIV-1 infected CLL-8 cells and HRPO-SPA.

A 1:10 dilution of plasma in PBS (diluted 1:25 in water) was added to a well on a slide. One well was used for positive control and another well for negative control. The test slide was incubated for about 40 minutes at 37°C. After incubation, the slide was washed three times in PBS, for five minutes each wash. HRPO-SPA, dissolved in 1 ml PBS, was added to the slide and the slide was incubated again for about 30 minutes at 37°C. Subsequently, the slide was washed three times in PBS. After washing, the slides were immersed in 30 mg AEC dissolved in 7.5 ml dimethyl formamide (DMF), plus 17.5 ml of 0.35M sodium acetate buffer, and 250 ul of 30% hydrogen peroxide for 3 minutes. The reaction was stopped by dipping the slide in water.

Coloration of the cell's membrane and cytoplasm, detectable by light microscopy, indicated a positive reaction. The positive reaction indicates the presence of HIV-1 antibodies in the test sample.

Example 8

This Example illustrates the specificity of the IE test kit.

TABLE II

| | Number of Sera | Positive Results | |
|---|---|---|---|
| | | IF | IE |
| AIDS Patients<br>Uninfected Persons | 30<br>30 | 30<br>0 | 30<br>0 |
| Positive Value | | 100% | 100% |

The date presented in Table II demonstrate that the specificity of the IE test kit is the same as that of the IF test. No false positive results were obtained.

Example 9

This Example illustrates the reproducibility of the conjugation of HRPO and SPA.

TABLE III

| No. of Sera | | Titer of HIV-1 Antibody* | | |
|---|---|---|---|---|
| | | **8901 | 8902 | 8903 |
| AIDS patients | 1 | 1:5120 | 1:5120 | 1:2560 |
| | 2 | 1:640 | 1:640 | 1:640 |
| | 3 | 1:640 | 1:640 | 1:640 |
| | 4 | 1:640 | 1:640 | 1:640 |
| | 5 | 1:160 | 1:160 | 1:160 |
| | 6 | 1:640 | 1:640 | 1:1280 |
| | 7 | 1:1280 | 1:1280 | 1:1280 |
| | 8 | 1:80 | 1:80 | 1:80 |
| | 9 | 1:1280 | 1:640 | 1:1280 |
| | 10 | 1:320 | 1:320 | 1:320 |
| Uninfected Persons | 11 | - | - | - |
| | 12 | - | - | - |
| | 13 | - | - | - |
| | 14 | - | - | - |
| | 15 | - | - | - |

* Titer of HIV-1 antibody for which a positive reaction was obtained.
** Lot number of conjugations

The data presented in Table III demonstrate that the conjugation of HRPO and SPA is highly reproducible. Highly reproducible HRPO-SPA conjugation is important to ensure accurate, reproducible results with the immuno-enzymatic test method. This measure safeguards against false negative results.

Example 10

This Example illustrates the reproducibility of the results obtained with IE test slides.

TABLE IV

| Numbers of Sera | | Titer of HIV-1 Antibody | | |
|---|---|---|---|---|
| | | *89001 | 89002 | 89003 |
| AIDS patients | 1 | 1:5120 | 1:5120 | 1:5120 |
| | 2 | 1:640 | 1:640 | 1:640 |
| | 3 | 1:640 | 1:640 | 1:640 |
| | 4 | 1:640 | 1:640 | 1:1280 |
| | 5 | 1:160 | 1:160 | 1:160 |
| | 6 | 1:640 | 1:640 | 1:640 |
| | 7 | 1:1280 | 1:1280 | 1:640 |
| | 8 | 1:80 | 1:80 | 1:80 |
| | 9 | 1:1280 | 1:1280 | 1:1280 |
| | 10 | 1:320 | 1:320 | 1:320 |
| Uninfected persons | 11 | - | - | - |
| | 12 | - | - | - |
| | 13 | - | - | - |
| | 14 | - | - | - |
| | 15 | - | - | - |

*Lot number of slides

The data presented in Table IV demonstrate that highly consistent results are obtained with different lots of IE test slides.

Example 11

This Example compares the reproducibility of IE test results with those of the IF test.

TABLE V

| No. of Sera | Titer of HIV-1 Antibody | | | | | |
|---|---|---|---|---|---|---|
| | IF | | | IE | | |
| | 1 | 2 | 3 | 1 | 2 | 3 |
| AIDS patients | 468 1:5120 | 1:5120 | 1:2560 | 1:20480 | 1:10240 | 1:20480 |
| | 618 1:640 | 1:640 | 1:640 | 1:1280 | 1:1280 | 1:1280 |
| | 623 1:640 | 1:640 | 1:640 | 1:1280 | 1:1280 | 1:1280 |
| | 630 1:160 | 1:160 | 1:160 | 1:160 | 1:160 | 1:160 |
| | 645 1:160 | 1:160 | 1:160 | 1:160 | 1:160 | 1:160 |
| | 652 1:1280 | 1:1280 | 1:1280 | 1:5120 | 1:5120 | 1:5120 |
| Uninfected persons | 9 - | - | - | - | - | - |
| | 21 - | - | - | - | - | - |

The data presented in Table V demonstrate that the reproducibility of the IE test is comparable to that of the IF test.

Example 12

This Example compares the sensitivity of the IE test with that of the IF test.

TABLE VI

| No. of Sera | Titer of HIV-1 Antibody | |
|---|---|---|
| | IF | IE |
| 468 | 1:5120 | 1:20480 |
| 539 | 1:640 | 1:1280 |
| 543 | 1:640 | 1:640 |
| 618 | 1:640 | 1:1280 |
| 623 | 1:640 | 1:1280 |
| 630 | 1:160 | 1:160 |
| 645 | 1:1280 | 1:5120 |
| 646 | 1:640 | 1:1280 |
| 652 | 1:1280 | 1:5120 |
| 663 | 1:80 | 1:80 |
| GMT | 642.69 | 1282.33 |

The data presented in Table VI demonstrate that the IE test is more sensitive than the IF test in most cases.

Example 13

This Example illustrates the stability of IE test slides.

9

TABLE VII

Slide Lot No. 89001

| No. of Sera | | Titer of HIV-1 Antibody | | | | | | | | |
|---|---|---|---|---|---|---|---|---|---|---|
| | | One Day | | 1 week | | 2 weeks | | 3 mo. | 6mo. | 1 yr. |
| | | 4 C | Room Temp | 4 C | Room Temp | 4 C | Room Temp | 4C | 4C | 4C |
| AIDS | 1 | 1:80 | 1:80 | 1:80 | 1:80 | 1:80 | 1:80 | 1:80 | 1:80 | 1:80 |
| | 2 | 1:320 | 1:320 | 1:320 | 1:320 | 1:320 | 1:320 | 1:320 | 1:320 | 1:320 |
| Patients | 3 | 1:640 | 1:640 | 1:640 | 1:640 | 1:640 | 1:640 | 1:640 | 1:640 | 1:640 |
| | 4 | 1:160 | 1:160 | 1:160 | 1:160 | 1:160 | 1:160 | 1:160 | 1:160 | 1:160 |
| | 5 | 1:640 | 1:640 | 1:640 | 1:640 | 1:640 | 1:640 | 1:640 | 1:640 | 1:640 |
| Unin- | 6 | - | - | - | - | - | - | - | - | - |
| fected | 7 | - | - | - | - | - | - | - | - | - |
| Persons | 8 | - | - | - | - | - | - | - | - | - |

Slide Lot No. 89002

| | | 4 C | Room Temp | 4 C | Room Temp | 4 C | Room Temp | 4C | 4C | 4C |
|---|---|---|---|---|---|---|---|---|---|---|
| AIDS | 1 | 1:80 | 1:80 | 1:80 | 1:80 | 1:80 | 1:80 | 1:80 | 1:80 | 1:80 |
| | 2 | 1:320 | 1:320 | 1:320 | 1:320 | 1:320 | 1:320 | 1:320 | 1:320 | 1:320 |
| Patients | 3 | 1:640 | 1:640 | 1:640 | 1:640 | 1:640 | 1:640 | 1:640 | 1:640 | 1:640 |
| | 4 | 1:160 | 1:160 | 1:160 | 1:160 | 1:160 | 1:160 | 1:160 | 1:160 | 1:160 |
| | 5 | 1:640 | 1:640 | 1:640 | 1:640 | 1:640 | 1:640 | 1:640 | 1:640 | 1:640 |
| Unin- | 6 | - | - | - | - | - | - | - | - | - |
| fected | 7 | - | - | - | - | - | - | - | - | - |
| Persons | 8 | - | - | - | - | - | - | - | - | - |

Slide Lot No. 89003

| | | 4 C | Room Temp | 4 C | Room Temp | 4 C | Room Temp | 4C | 4C | 4C |
|---|---|---|---|---|---|---|---|---|---|---|
| AIDS | 1 | 1:80 | 1:80 | 1:80 | 1:80 | 1:80 | 1:80 | 1:80 | 1:80 | 1:80 |
| | 2 | 1:320 | 1:320 | 1:320 | 1:320 | 1:320 | 1:320 | 1:320 | 1:320 | 1:320 |
| Patients | 3 | 1:640 | 1:640 | 1:640 | 1:640 | 1:640 | 1:640 | 1:640 | 1:640 | 1:640 |
| | 4 | 1:160 | 1:160 | 1:160 | 1:160 | 1:160 | 1:160 | 1:160 | 1:160 | 1:160 |
| | 5 | 1:640 | 1:640 | 1:640 | 1:640 | 1:640 | 1:640 | 1:640 | 1:640 | 1:640 |
| Unin- | 6 | - | - | - | - | - | - | - | - | - |
| fected | 7 | - | - | - | - | - | - | - | - | - |
| Persons | 8 | - | - | - | - | - | - | - | - | - |

The data presented in Table VII demonstrate that the IE test slides are stable at 4°C or room temperature for a period of one year. The ability to store IE test slides either at 4°C or at room temperature for an extended period of time is an advantage over storage requirements and stability of components of other test methods.

Example 14

This Example illustrates the stability of the IE test conjugates.

TABLE VIII

Conjugate Lot No. 8901

| No. of Sera | | Titer of HIV-1 Antibody | | | | | | | | |
|---|---|---|---|---|---|---|---|---|---|---|
| | | One Day | | 1 week | | 2 weeks | | 3 mo. | 6mo. | 1 yr. |
| | | 4 C | Room Temp | 4 C | Room Temp | 4 C | Room Temp | 4C | 4C | 4C |
| AIDS | 1 | 1:80 | 1:80 | 1:80 | 1:80 | 1:80 | 1:80 | 1:80 | 1:80 | 1:80 |
| | 2 | 1:320 | 1:320 | 1:320 | 1:320 | 1:320 | 1:320 | 1:320 | 1:320 | 1:320 |
| Patients | 3 | 1:640 | 1:640 | 1:640 | 1:640 | 1:640 | 1:640 | 1:640 | 1:640 | 1:640 |
| | 4 | 1:160 | 1:160 | 1:160 | 1:160 | 1:160 | 1:160 | 1:160 | 1:160 | 1:160 |
| | 5 | 1:640 | 1:640 | 1:640 | 1:640 | 1:640 | 1:640 | 1:640 | 1:640 | 1:640 |
| Unin- | 6 | – | – | – | – | – | – | – | – | – |
| fected | 7 | – | – | – | – | – | – | – | – | – |
| Persons | 8 | – | – | – | – | – | – | – | – | – |

Conjugate Lot No. 8902

| AIDS | 1 | 1:80 | 1:80 | 1:80 | 1:80 | 1:80 | 1:80 | 1:80 | 1:80 | 1:80 |
|---|---|---|---|---|---|---|---|---|---|---|
| | 2 | 1:320 | 1:320 | 1:320 | 1:320 | 1:320 | 1:320 | 1:320 | 1:320 | 1:320 |
| Patients | 3 | 1:640 | 1:640 | 1:640 | 1:640 | 1:640 | 1:640 | 1:640 | 1:640 | 1:640 |
| | 4 | 1:160 | 1:160 | 1:160 | 1:160 | 1:160 | 1:160 | 1:160 | 1:160 | 1:160 |
| | 5 | 1:640 | 1:640 | 1:640 | 1:640 | 1:640 | 1:640 | 1:640 | 1:640 | 1:640 |
| Unin- | 6 | – | – | – | – | – | – | – | – | – |
| fected | 7 | – | – | – | – | – | – | – | – | – |
| Persons | 8 | – | – | – | – | – | – | – | – | – |

Conjugate Lot No. 8903

| AIDS | 1 | 1:80 | 1:80 | 1:80 | 1:80 | 1:80 | 1:80 | 1:80 | 1:80 | 1:80 |
|---|---|---|---|---|---|---|---|---|---|---|
| | 2 | 1:320 | 1:320 | 1:320 | 1:320 | 1:320 | 1:320 | 1:320 | 1:320 | 1:320 |
| Patients | 3 | 1:640 | 1:640 | 1:640 | 1:640 | 1:640 | 1:640 | 1:640 | 1:640 | 1:640 |
| | 4 | 1:160 | 1:160 | 1:160 | 1:160 | 1:160 | 1:160 | 1:160 | 1:160 | 1:160 |
| | 5 | 1:640 | 1:640 | 1:640 | 1:640 | 1:640 | 1:640 | 1:640 | 1:640 | 1:640 |
| Unin- | 6 | – | – | – | – | – | – | – | – | – |
| fected | 7 | – | – | – | – | – | – | – | – | – |
| Persons | 8 | – | – | – | – | – | – | – | – | – |

The data in Table VIII demonstrate the stability of conjugates resulting from the IE test at 4°C or room temperature for a period of one year. The highly stable nature of the IE test conjugates allows storage of the test slide results for an extended period of time, should confirmation of test results be required.

Example 15

This Example compares the IE test with the Western blot.

TABLE IX

| | | Western Blot Test | | Total |
|---|---|---|---|---|
| | | Positive | Negative | |
| IE | Positive | 222 | 7 | 223 |
| Test | Negative | 0 | 353 | 353 |
| Total | | 222 | 360 | 582 |

Comparison of the IE test with the Western blot indicates that the IE test is 100% sensitive, with a specificity of 98.1%. The predictive value of a positive test (PPV) is 96.9%, with 1.9% false positive results, whereas the predictive value of a negative test (NPV) is 100%, with no false negative results. The Youden Index, an epidemiological factor, is 0.98.

Example 16

This Example compares the sensitivity of the IE test strip with that of other test methods.

The HIV-1 antigen test strip kit was compared with the test methods of Genetic Systems and Organon-Tekneka at the Center for Disease Control (CDC) in Atlanta, Georgia. The most stringent test to grade AIDS tests was used.

The grading test consists of a collection of blood samples taken from a man infected with AIDS virus, including a sample taken on the day believed to be the day of infection and several additional, serial samples, taken every few days after that. The results of the comparison testing are summarized in Table X.

Table X

| Date* | Test Method for HIV-1 Antigen | | Test Method for HIV-1 Antibody | | |
|---|---|---|---|---|---|
| | Abbott | DuPont | HIV-1 Antigen Test Strip (1/20 dilution) | Organon-Technika (straight sample) | Genetic Systems (straight sample) |
| 11-14-88 | + | + | - | - | - |
| 11-17-88 | + | + | - | - | - |
| 11-21-88 | + | + | - | - | - |
| 11-25-88 | + | + | + | " + " | ± |
| 11-28-88 | ± | + | + | + | + |
| 12-02-88 | ± | - | + | + | + |
| 12-08-88 | - | - | + | + | + |
| 12-12-88 | - | - | + | + | + |
| 12-16-88 | - | - | + | + | + |
| 12-19-88 | - | - | + | + | + |
| 04-16-89 | - | - | + | + | + |

*date of collection from man infected with AIDS virus.

The HIV-1 antigen test strip was able to detect antibodies to HIV-1 in a 1/20 dilution of the blood sample taken 11 days post-infection. The Organon-Technika test method detected antibodies in the undiluted, straight sample. However, the level of antibodies in the straight sample was only marginally detectable with the test method of Genetic Systems. The fact that the HIV-1 antigen test strip of the present invention was able to detect antibodies in a 1/20 dilution indicates the high degree of sensitivity attainable with the test strip. Given this high degree of sensitivity, it is believed that the test strip would have detected antibodies in a straight, undiluted blood sample taken prior to 11 days post-infection, thereby allowing for even earlier detection of antibodies in terms of absolute detection and relative to the most sensitive commercially available rapid detection test. In short, the present invention provides a most sensitive test for the AIDS virus.

The HIV-1 antigen test strip was compared to the ELISA test at New Mexico State University. The results of the comparison are summarized in Table XI.

Table XI

| Serum Sample No. | ELISA (straight sample) | HIV-1 Antigen Test Strip (1/20 dilution) |
|---|---|---|
| 1, 23* | + | + |
| 2, 15 | + | + |
| 4, 24 | - | - |
| 5, 19 | - | - |
| 7, 20 | + | + |
| 10, 17 | + | + |
| 11, 16 | + | + |
| 12, 22 | + | + |
| 14, 21 | + | + |
| 3, 18 | + | + |
| 4338 | + | + |
| 117625 | + | + |
| 3590 | + | + |
| 6 | + | + |
| positive control | N/A | + |
| negative control | N/A | - |

*two samples from same patient; blind-labeled.

The results obtained with the dip strip are comparable to those obtained with the ELISA test, which is the most sensitive test for the determination of the AIDS virus and for other viruses as well. The present invention thus provides a simple, rapid, highly sensitive and accurate test for the determination of the presence of a virus in blood and blood components.

The virus HIV-1Ac has been deposited with the American Type Culture Collection under No. ATCC VR2338. The CM-1 and CLL-8 cells have been deposited with the American Type Culture Collection under the following Nos.:

CM-1 under ATCC CRL 10876

CLL-8 (designated as C11-8) under ATCC CRL 10877

The plasmid pBV221 (designated as pBV221-T) has been deposited with the American Type Culture Collection under No. ATCC 75106.

## Claims

1. An immuno-enzymatic method for the detection of antibodies to a virus under test in a sample comprising:

(a) contacting the sample with T-cells, said T-cells having been infected with said virus and activated with an inducer capable of promoting intracellular accumulation of the virus and cell surface presentation of viral antigens, said cells having been fixed to a support means;

(b) contacting said T-cells with a conjugate capable of binding to said antibodies and capable of inducing a color change in an indicator,

(c) contacting said T-cells that have been contacted with said conjugate with an indicator for said conjugate; and

(d) detecting a change in said indicator from colorless to colored by observation with a light microscope, said color change being indicative of the presence in said sample of antibodies to the virus under test.

2. An immuno-enzymatic method according to claim 1 wherein said T-cells are selected from the group consisting of CLL-8 cells and CM-1 cells.

3. An immuno-enzymatic method according to claim 1 wherein said T-cells are infected with a virus selected from the group consisting of HIV-1, HIV-2, HTLV-1, and SIV-III.

4. An immuno-enzymatic method according to claim 3 wherein the HIV-1 virus is derived from the isolate HIV-1-AC.

5. An immuno-enzymatic method according to claim 1, wherein said inducer comprises 5 ng/ml cortisone, 5-20 ng/ml TPA, and 1-4 mM butyric acid in a culture medium.

6. An immuno-enzymatic method according to claim 5, wherein said inducer consists essentially of 5 ng/ml cortisone, 5 ng/ml TPA and 2 mM butyric acid in a culture medium.

7. An immuno-enzymatic method according to claim 1 wherein the conjugate is selected from the group consisting of Horseradish peroxidase (HRPO)-Staphylococcus protein A (SPA), HRPO - anti-viral IgG antibody, phosphatase-SPA, and phosphatase-anti-viral IgG antibody.

8. An immuno-enzymatic method according to claim 7 wherein the indicator for HRPO is 3-amino-9-ethylcarbazole (AEC).

9. An immuno-enzymatic method according to claim 1 wherein the viral antibodies to be detected are selected from the group consisting of HIV-1, HIV-2, HTLV-1, and SIV-III.

10. An immuno-enzymatic method for the detection of HIV-1 antibodies in blood or blood components comprising:
(a) contacting the blood or blood components with cells selected from the group consisting of CLL-8 cells and CM-1 cells, said cells having been infected with HIV-1 and activated with an inducer, said inducer comprising 5 ng/ml TPA, 5 ng/ml cortisone and 2mM butyric acid in a culture medium, said cells having been fixed to a support means;
(b) contacting said cells with HRPO-conjugated SPA;
(c) contacting said cells of step (b) with AEC;
(d) detecting a change in AEC from colorless to pink as observed with the light microscope, said color change being indicative of the presence in said blood or blood components of antibodies to the virus under test.

11. An immuno-enzymatic method according to claim 10 wherein the HIV-1 virus is derived from the isolate HIV-1-AC.

12. An immuno-enzymatic method for the detection of antibodies to Ebola virus in a sample comprising:
(a) contacting the sample with Vero cells, said cells having been infected with said virus, said cells having been fixed to a support means;
(b) contacting said Vero cells with a conjugate capable of binding to said antibodies and capable of inducing a color change in an indicator,
(c) contacting said Vero cells that have been contacted with said conjugate with an indicator for said conjugate; and
(d) detecting a change in said indicator from colorless to colored by observation with the light microscope, said color change being indicative of the presence in said sample of antibodies to Ebola virus.

13. An immuno-enzymatic method according to claim 12 wherein the conjugate is selected from the group consisting of Horseradish peroxidase (HRPO)-Staphylococcus protein A (SPA), HRPO - anti-viral IgG antibody, phosphatase-SPA, and phosphatase-anti-viral IgG antibody.

14. An immuno-enzymatic method according to claim 13 wherein the indicator for HRPO is 3-amino-9-ethylcarbazole (AEC).

15. An immuno-enzymatic method for the detection of antibodies to Epstein-Barr virus in a sample comprising:
(a) contacting the sample with B-cells, said B-cells having been infected with said virus and activated with an inducer capable of promoting intracellular accumulation of the virus and cell surface presentation of viral antigens, said cells having been fixed to a support means;
(b) contacting said B-cells with a conjugate capable of binding to said antibodies and capable of

14

inducing a color change in an indicator,

(c) contacting said B-cells that have been contacted with said conjugate with an indicator for said conjugate; and

(d) detecting a change in said indicator from colorless to colored by observation with the light microscope, said color change being indicative of the presence in said sample of antibodies to Epstein-Barr virus.

16. An immuno-enzymatic method according to claim 15 wherein said B-cells are Raji cells.

17. An immuno-enzymatic method according to claim 15, wherein said inducer comprises 5 ng/ml cortisone, 5-20 ng/ml TPA, and 1-4 mM butyric acid in a culture medium.

18. An immuno-enzymatic method according to claim 17, wherein said inducer consists essentially of 5 ng/ml cortisone, 5 ng/ml TPA and 2 mM butyric acid in a culture medium.

19. An immuno-enzymatic method according to claim 15 wherein the conjugate is selected from the group consisting of Horseradish peroxidase (HRPO)-Staphylococcus protein A (SPA), HRPO - anti-viral IgG antibody, phosphatase-SPA, and phosphatase-anti-viral IgG antibody.

20. An immuno-enzymatic method according to claim 19 wherein the indicator for HRPO is 3-amino-9-ethylcarbazole (AEC).

21. An immuno-enzymatic method for the detection of antibodies to Epstein-Barr virus in a sample comprising:

(a) contacting the sample with B95-8 cells, said cells having been infected with said virus, said cells having been fixed to a support means;

(b) contacting said B95-8 cells with a conjugate capable of binding to said antibodies and capable of inducing a color change in an indicator,

(c) contacting said B95-8 cells that have been contacted with said conjugate with an indicator for said conjugate; and

(d) detecting a change in said indicator from colorless to colored by observation with the light microscope, said color change being indicative of the presence in said sample of antibodies to Epstein-Barr virus.

22. An immuno-enzymatic method according to claim 21 wherein the conjugate is selected from the group consisting of Horseradish peroxidase (HRPO)-Staphylococcus protein A (SPA), HRPO - anti-viral IgG antibody, phosphatase-SPA, and phosphatase-anti-viral IgG antibody.

23. An immuno-enzymatic method according to claim 22 wherein the indicator for HRPO is 3-amino-9-ethylcarbazole (AEC).

24. A method of establishing a high intracellular concentration of virus and increased cell surface presentation of viral antigens in a cell line comprising:

(a) infecting the cells with virus; and

(b) activating the infected cells with an inducer capable of promoting intracellular accumulation of virus and cell surface presentation of viral antigens.

25. A method according to claim 24 wherein the cells are selected from the group consisting of T-cells and B-cells.

26. A method according to claim 25 wherein the T-cells are selected from the group consisting of CLL-8 cells and CM-1 cells.

27. A method according to claim 25 wherein the B-cells are Raji cells.

28. A method according to claim 26 wherein said cells are infected with a virus selected from the group consisting of HIV-1, HIV-2, HTLV-1, and SIV-III.

**29.** A method according to claim 28 wherein the HIV-1 virus is derived from the isolate HIV-1-AC.

**30.** A method according to claim 27 wherein the Raji cells are infected with Epstein-Barr virus.

**31.** A method according to claim 24 wherein said inducer comprises 5 ng/ml cortisone, 5-20 ng/ml TPA and 1-4 mM butyric acid in a culture medium.

**32.** A method according to claim 31 wherein said inducer consists essentially of 5 ng/ml cortisone, 5 ng/ml TPA and 2 mM butyric acid in a culture medium.

**33.** A method of establishing a high intracellular concentration of HIV-1 virus and increased cell surface presentation of HIV-1 antigens in cells selected from the group consisting of CLL-8 cells and CM-1 cells, comprising:
(a) infecting said cells with HIV;
(b) activating the HIV-1-infected cells with an inducer comprising 5 ng/ml TPA, 5ng/ml cortisone and 2mM butyric acid to promote intracellular accumulation of HIV-1 and cell surface presentation of HIV-1 antigens.

**34.** A method of establishing a high intracellular concentration of HIV-1 virus and increased cell surface presentation of HIV-1 antigens according to claim 33 wherein the HIV-1 virus is derived from the isolate HIV-1-AC.

**35.** A cell line designated CLL-8, established from a T-cell leukemia patient.

**36.** A cell line designated CM-1, established from T-cells of a comatose patent.

**37.** An isolate of HIV-1 virus designated HIV-1-AC, obtained from an American AIDS patient in China.

**38.** An inducer capable of promoting intracellular accumulation of a virus and cell surface presentation of viral antigens comprising 5ng/ml cortisone, 5ng/ml TPA and 2mM butyric acid in culture medium.

**39.** An immuno-enzymatic test kit for the detection of antibodies to a virus under test in a test sample comprising:
(a) support means having fixed thereto T-cells, at least a portion of said T-cells having been infected with said virus and activated with an inducer capable of promoting intracellular accumulation of the virus and cell surface presentation of viral antigens;
(b) a first container having a conjugate capable of binding to said antibodies and capable of inducing a color change in an indicator therein;
(c) a second container having an indicator for the conjugate therein.

**40.** An immuno-enzymatic test kit according to claim 39, wherein the T-cells are selected from the group consisting of CLL-8 cells and CM-1 cells.

**41.** An immuno-enzymatic test kit according to claim 39 wherein a portion of said T-cells are infected with a virus selected from the group consisting of HIV-1, HIV-2, HTLV-1, and SIV-III.

**42.** An immuno-enzymatic test kit according to claim 41 wherein the HIV-1 virus is derived from the isolate designated HIV-1-AC.

**43.** An immuno-enzymatic test kit according to claim 39 wherein the conjugate is selected from the group consisting of HRPO-SPA, HRPO-anti-viral IgG antibody, alkaline phosphatase-SPA and alkaline phosphatase-anti-viral IgG antibody.

**44.** An immuno-enzymatic test kit according to claim 43 wherein the indicator for HRPO is AEC.

**45.** An immuno-enzymatic test kit according to claim 39 wherein the viral antibodies to be detected are from the group consisting essentially of HIV-1, HIV-2, HTLV-1, and SIV-III.

EP 0 495 465 A2

46. An immuno-enzymatic test kit for the detection of HIV-1 antibodies in blood or blood components comprising:

(a) support means having fixed thereto cells selected from the group consisting of CLL-8 cells and CM-1 cells, said cells having been infected with HIV-1 and activated with an inducer, comprising 5 ng/ml cortisone, 5 ng/ml TPA and 2mM butyric acid;

(b) a first container having a HRPO-SPA conjugate capable of binding to said antibodies and capable of inducing a color change in the indicator AEC therein;

(c) a second container having the AEC indicator for HRPO therein.

47. An immuno-enzymatic test kit according to claim 46 wherein the HIV-1 virus is derived from the isolate HIV-1-AC.

48. An immuno-enzymatic test kit for the detection of antibodies to Ebola virus in a test sample comprising:

(a) support means having fixed thereto Vero cells, at least a portion of said Vero cells having been infected with said virus;

(b) a first container having a conjugate capable of binding to said antibodies and capable of inducing a color change in an indicator therein;

(c) a second container having an indicator for the conjugate therein.

49. An immuno-enzymatic test kit according to claim 48 wherein the conjugate is selected from the group consisting of HRPO-SPA, HRPO-anti-viral IgG antibody, phosphatase-SPA and phosphatase-anti-viral IgG antibody.

50. An immuno-enzymatic test kit according to claim 49 wherein the indicator for HRPO is AEC.

51. An immuno-enzymatic test kit for the detection of antibodies to Epstein-Barr virus in a test sample comprising:

(a) support means having fixed thereto B-cells, at least a portion of said B-cells having been infected with said virus and activated with an inducer capable of promoting intracellular accumulation of the virus and cell surface presentation of viral antigens;

(b) a first container having a conjugate capable of binding to said antibodies and capable of inducing a color change in an indicator therein;

(c) a second container having an indicator for the conjugate therein.

52. An immuno-enzymatic test kit according to claim 51, wherein the B-cells are Raji cells.

53. An immuno-enzymatic test kit according to claim 51 wherein the conjugate is selected from the group consisting of HRPO-SPA, HRPO-anti-viral IgG antibody, phosphatase-SPA and phosphatase-anti-viral IgG antibody.

54. An immuno-enzymatic test kit according to claim 53 wherein the indicator for HRPO is AEC.

55. An immuno-enzymatic test kit for the detection of antibodies to Epstein-Barr virus in a test sample comprising:

(a) support means having fixed thereto B-cells, at least a portion of said B-cells having been infected with said virus;

(b) a first container having a conjugate capable of binding to said antibodies and capable of inducing a color change in an indicator therein;

(c) a second container having an indicator for the conjugate therein.

56. An immuno-enzymatic test kit according to claim 55, wherein the B-cells are B95-8 cells.

57. An immuno-enzymatic test kit according to claim 55 wherein the conjugate is selected from the group consisting of HRPO-SPA, HRPO-anti-viral IgG antibody, phosphatase-SPA and phosphatase-anti-viral IgG antibody.

58. An immuno-enzymatic test kit according to claim 57 wherein the indicator for HRPO is AEC.

17

59. An immuno-enzymatic test strip suitable for the detection of antibodies to a virus comprising a substrate, said substrate carrying thereon T-cells, said T-cells having been infected with virus and activated with an inducer, said inducer capable of promoting intracellular accumulation of the virus and cell surface presentation of viral antigens.

60. An immuno-enzymatic test strip according to claim 59 wherein the T-cells are selected from the group consisting of CLL-8 cells and CM-1 cells.

61. An immuno-enzymatic test strip according to claim 59 wherein the infecting virus is from the group consisting essentially of HIV-1, HIV-2, HTLV-1, and SIV-III.

62. An immuno-enzymatic test strip according to claim 61 wherein the HIV-1 virus is derived from the isolate HIV-1-AC.

63. An immuno-enzymatic test strip suitable for the detection of antibodies to Ebola virus comprising a substrate, said substrate carrying thereon Vero cells, said Vero cells having been infected with Ebola virus.

64. An immuno-enzymatic test strip suitable for the detection of antibodies to Epstein-Barr virus comprising a substrate, said substrate carrying thereon B-cells, said cells having been infected with Epstein-Barr virus and activated with an inducer, said inducer capable of promoting intracellular accumulation of the virus and cell surface presentation of viral antigens.

65. An immuno-enzymatic test strip according to claim 64 wherein the B-cells are Raji cells.

66. An immuno-enzymatic test strip suitable for the detection of antibodies to Epstein-Barr virus comprising a substrate, said substrate carrying thereon B-cells, said cells having been infected with Epstein-Barr virus.

67. An immuno-enzymatic test strip according to claim 66 wherein the B-cells are B95-8 cells.

68. An immuno-enzymatic test strip suitable for the detection of antibodies to a virus comprising a substrate, said substrate carrying thereon the virus under test.

69. An immuno-enzymatic test strip according to claim 68 wherein the virus under test is from the group consisting essentially of HIV-1, HIV-2, HTLV-1, SIV-III, Ebola and Epstein-Barr viruses.

70. An immuno-enzymatic test strip according to claim 69 wherein the HIV-1 virus is derived from the isolate HIV-1-AC.

71. An immuno-enzymatic test strip suitable for the detection of antibodies to a virus comprising a substrate, said substrate carrying thereon antigens from the virus under test.

72. An immuno-enzymatic test strip according to claim 71 wherein the antigens from the virus under test are from the group consisting of HIV-1, HIV-2, HTLV-1, SIV-III, Ebola and Epstein-Barr viruses.

73. An immuno-enzymatic test strip according to claim 71 wherein the virus under test is HIV-1 and the antigen from the HIV-1 virus is gp41.

74. An immuno-enzymatic test-strip kit for the detection of antibodies to a virus under test comprising:
(a) immuno-enzymatic test strip suitable for the detection of antibodies to a virus comprising a substrate, said substrate carrying thereon antigens from the virus under test;
(b) a first container having a conjugate capable of binding to said antibodies and capable of inducing a color change in an indicator therein; and
(c) a second container having an indicator for the conjugate therein.

75. An immuno-enzymatic test-strip kit for the detection of antibodies to HIV-1 virus comprising:
(a) an immuno-enzymatic test strip comprising a substrate having gp41 adsorbed thereon;

18

(b) a first container having HRPO-SPA conjugate capable of binding to said antibodies therein; and
(c) a second container having AEC indicator for HRPO therein.

**76.** The recombinant vector pBY221, which contains the HIV-1 gene for gp41.

**77.** An E. coli cell line transformed with the recombinant vector pBV221, which contains the HIV-1 gene for gp41.

**78.** The production of gp41 protein from an E. coli cell line transformed with the recombinant vector pBV221, which contains the HIV-1 gene for gp41.

FIG. 1